# EUROPEAN PATENT SPECIFICATION

(11) **EP 3 013 929 B1**
(45) Date of publication and mention of the grant of the patent: **22.08.2018**
(21) Application number: 14740061.8
(22) Date of filing: 23.06.2014
(51) Int. Cl.: C11B 1/10, C11C 3/10, A23D 9/02, C11B 1/02

(54) **DIRECT METHOD OF PRODUCING FATTY ACID ESTERS FROM MICROBIAL BIOMASS**
DIREKTES VERFAHREN ZUR HERSTELLUNG VON FETTSÄUREESTERN AUS MIKROBIELLER BIOMASSE
PROCÉDÉ DIRECT DE PRODUCTION D'ESTERS D'ACIDES GRAS À PARTIR DE BIOMASSE MICROBIENNE

(30) Priority: 25.06.2013 US 201361838944 P
(43) Date of publication of application: 04.05.2016
(73) Proprietor: Shell Internationale Research Maatschappij B.V., 2596 HR The Hague (NL)
(72) Inventor: WANG, Pen-Chung, Houston, Texas 77079 (US); WEIDER, Paul Richard, Houston, Texas 77095 (US); BLACKBOURN, Robert Lawrence, Houston, Texas 77095 (US); ALISHUSKY, Joseph James, Houston, Texas 77095 (US)
(74) Representative: Shell Legal Services IP
(86) International application number: PCT/US2014/043561
(87) International publication number: WO 2014/209830

(56) References cited:
- EP-A1- 2 333 093
- EP-A1- 2 450 425
- EP-A2- 2 280 062
- WO-A1-2013/082141

## Description

### Field of the Invention

The invention relates to a process for producing fatty acid esters from microbial biomass.

### Background of the Invention

Microorganisms such as fungi, yeast, bacteria, and algae have ability to produce lipids. In a typical process to produce fatty acid esters for use as biodiesel from lipids, lipids are first recovered from microbial biomass and then acid- or alkali catalyzed (such as sulfuric acid or sodium hydroxide) to transesterify the lipids to fatty acid esters.

Lipids constitute a broad group of naturally occurring molecules that include fats, waxes, sterols, fat-soluble vitamins (such as vitamins A, D, E, and K), monoglycerides, diglycerides, triglycerides, phospholipids, and others. The main biological functions of lipids include energy storage, as structural components of cell membranes, and as important signaling molecules. Lipid is generally accumulated in microbial cell. Therefore, there have been practiced a variety of methods to extract lipids from microbial cells endowed with lipid-producing ability. To release lipids from source material, it might be necessary to destruct cell walls prior to lipid extraction. The disruption may occur physically, enzymatically and/or chemically. Preferably, cell disruption is performed by mechanical means. Several methods have been used for the physical disruption of cells, including homogenization, sonication, freeze/thaw, extrusion, and mechanical grinding. However, these methods require quite a long time to recover a sufficient amount of lipids and therefore, efficient extraction cannot be performed. For example, homogenization of wet microbial biomass may create emulsions which make the subsequent extraction step difficult.

Therefore, it is desirable to develop a more cost effective, efficient method of producing fatty acid esters from microbial biomass for use as biodiesel. WO2013/2013082141 describes a method for obtaining lipids from microbial biomass by treating the biomass with a solution containing at least one a-hydroxysulfonic acid to extract and recover liposoluble components.

### Summary of the Invention

It has been found that the microbial oil extraction and transesterification can be combined into one through an in situ or direct transesterification process of the invention.

In an embodiment, a method of producing a fatty acid ester is provided comprising: (a) providing a microbial biomass; (b) contacting the microbial biomass with a solution containing an alcohol and at least one α-hydroxysulfonic acid thereby producing acid-treated biomass containing at least one fatty acid ester; and (c) recovering said fatty acid ester from the acid-treated biomass.

In yet another embodiment, a method of producing a fatty acid ester is provided comprising: (a) providing a microbial biomass; (b) contacting the microbial biomass with a solution containing an alcohol and at least one α-hydroxysulfonic acid thereby producing acid-treated biomass containing at least one fatty acid ester; (c) recovering said fatty acid ester from the acid-treated biomass; and (d) removing the α-hydroxysulfonic acid in its component form from the acid-treated biomass by heating and/or reducing pressure to produce an acid-removed product containing acid-treated biomass substantially free of the α-hydroxysulfonic acid.

In another embodiment, a method comprises recycling the removed α-hydroxysulfonic acid to step (b) as components or in its recombined form.

In yet another embodiment, a composition is provided comprising (a) a microbial biomass containing at least one lipid, (b) at least one α-hydroxysulfonic acid, (c) an alcohol, and (d) water. In yet further embodiment, a composition is provided comprising (a) biomass residue, (b) an alcohol, (c) at least one α-hydroxysulfonic acid, (d) water, and (e) at least one fatty acid alkyl ester.

The features and advantages of the invention will be apparent to those skilled in the art. While numerous changes may be made by those skilled in the art, such changes are within the spirit of the invention.

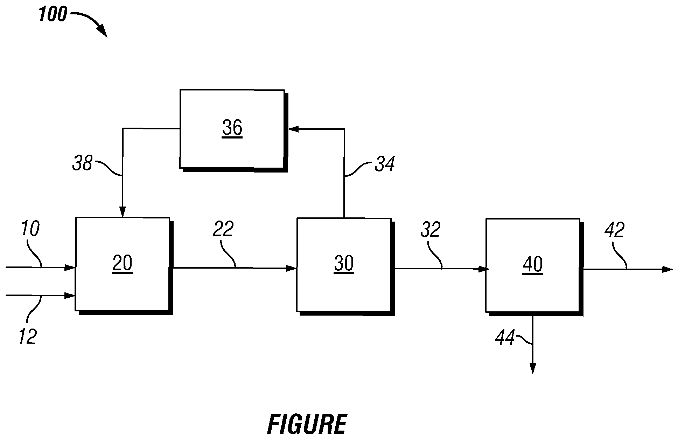

### Brief Description of the Drawing

This drawing illustrates certain aspects of some of the embodiments of the invention, and should not be used to limit or define the invention.

The Figure schematically illustrates a block flow diagram of an embodiment of the treatment process of this invention.

### Detailed Description of the Invention

It has been found that by use of α-hydroxysulfonic acid microbial biomass can be converted *in-situ* to biodiesel in a single step by disrupting microbial biomass cells, releasing liposoluble components and catalyzing the conversion to fatty acid esters (fatty acid alkyl esters) in the presence of alcohols. The α-hydroxysulfonic acid is effective for destruction of microbial cell walls and catalyzing the conversion improving the yield of fatty acid esters from the microbial biomass. Further, the α-hydroxysulfonic acid is reversible to readily removable and recyclable materials nor form emulsions such as by homogenization at high pressure.

Microorganisms containing lipids in the microbial cells like microbial biomass can be treated by the present process. Microbial biomass may be grown photosynthetically or by fermentation. Microbial biomass may include, for example, microalgae, yeast, fungi or bacteria.

The alpha-hydroxysulfonic acids of the general formula 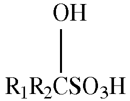 where R₁ and R₂ are individually hydrogen or hydrocarbyl with up to about 9 carbon atoms that may or may not contain oxygen can be used in the treatment of the instant invention. The alpha-hydroxysulfonic acid can be a mixture of the aforementioned acids. The acid can generally be prepared by reacting at least one carbonyl compound or precursor of carbonyl compound (e.g., trioxane and paraformaldehyde) with sulfur dioxide or precursor of sulfur dioxide (e.g., sulfur and oxidant, or sulfur trioxide and reducing agent) and water according to the following general equation 1. 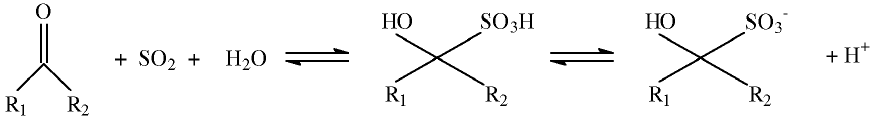 where R₁ and R₂ are individually hydrogen or hydrocarbyl with up to about 9 carbon atoms or a mixture tehreof.

Illustrative examples of carbonyl compounds useful to prepare the alpha-hydroxysulfonic acids used in this invention are found where
R₁=R₂=H (formaldehyde)
R₁=H, R₂=CH₃ (acetaldehyde)
R₁=H, R₂=CH₂CH₃ (propionaldehyde)
R₁=H, R₂= CH₂CH₂CH₃ (n-butyraldehyde)R₁=H, R₂=CH(CH₃)₂ (i-butyraldehyde)
R₁=H, R₂= CH₂OH (glycolaldehyde)
R₁=H, R₂= CHOHCH₂OH (glyceraldehdye)
R1=H, R2= C(=O)H (glyoxal) 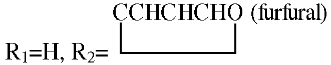 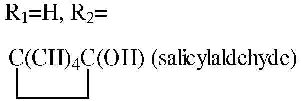 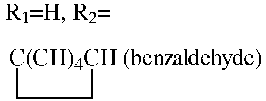
R₁=R₂=CH₃ (acetone)
R₁=CH₂OH, R₂=CH₃ (acetol)
R₁=CH₃, R₂=CH₂CH₃ (methyl ethyl ketone)
R₁=CH₃, R₂=CHC(CH₃)₂ (mesityl oxide)
R₁=CH₃, R₂=CH₂CH(CH₃)₂ (methyl i-butyl ketone)
R₁, R₂=(CH₂)₅ (cyclohexanone) or
R₁=CH₃, R₂=CH₂Cl (chloroacetone)

The carbonyl compounds and its precursors can be a mixture of compounds described above. For example, the mixture can be a carbonyl compound or a precursor such as, for example, trioxane which is known to thermally revert to formaldehyde at elevated temperatures or an alcohol that maybe converted to the aldehyde by dehydrogenation of the alcohol to an aldehyde by any known methods. An example of such a conversion to aldehyde from alcohol is described below. An example of a source of carbonyl compounds maybe a mixture of hydroxyacetaldehyde and other aldehydes and ketones produced from fast pyrolysis oil such as described in "Fast Pyrolysis and Bio-oil Upgrading, Biomass-to-Diesel Workshop", Pacific Northwest National Laboratory, Richland, Washington, September 5-6, 2006. The carbonyl compounds and its precursors can also be a mixture of ketones and/or aldehydes with or without alcohols that may be converted to ketones and/or aldehydes, preferably in the range of 1 to 7 carbon atoms.

The preparation of α-hydroxysulfonic acids by the combination of an organic carbonyl compounds, SO₂ and water is a general reaction and is illustrated in equation 2 for acetone. 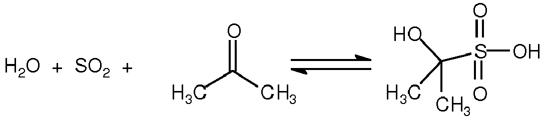 The α-hydroxysulfonic acids appear to be as strong as, if not stronger than, HCl since an aqueous solution of the adduct has been reported to react with NaCl freeing the weaker acid, HCl (see US 3,549,319). The reaction in equation 1 is a true equilibrium, which results in facile reversibility of the acid. That is, when heated, the equilibrium shifts towards the starting carbonyl, sulfur dioxide, and water (component form). If the volatile components (e.g. sulfur dioxide) is allowed to depart the reaction mixture via vaporization or other methods, the acid reaction completely reverses and the solution becomes effectively neutral. Thus, by increasing the temperature and/or lowering the pressure, the sulfur dioxide can be driven off and the reaction completely reverses due to Le Châtelier's principle, the fate of the carbonyl compound is dependant upon the nature of the material employed. If the carbonyl is also volatile (e.g. acetaldehyde), this material is also easily removed in the vapor phase. Carbonyl compounds such as benzaldehyde, which are sparingly soluble in water, can form a second organic phase and be separated by mechanical means. Thus, the carbonyl can be removed by conventional means, e.g., continued application of heat and/or vacuum, steam and nitrogen stripping, solvent washing, centrifugation, etc.. Therefore, the formation of these acids is reversible in that as the temperature is raised, the sulfur dioxide and/or aldehyde and/or ketone can be flashed from the mixture and condensed or absorbed elsewhere in order to be recycled. It has been found that these reversible acids, which are approximately as strong as strong mineral acids, are effective in disrupting cells of microorganisms and catalyzing the transesterification of lipids to fatty acid esters in the presence of alcohol. We have found that these treatment increase permeability of a solvent to the cells and the extraction efficiency of the lipids while catalyzing the transesterification reaction, thus facilitating fatty acid ester (fatty acid alkyl ester) production and recovery. Additionally, since the acids are effectively removed from the reaction mixture following treatment, neutralization with base to complicate downstream processing is substantially avoided. The ability to reverse and recycle these acids also allows the use of higher concentrations than would otherwise be economically or environmentally practical.

It has been found that the position of the equilibrium given in equation 1 at any given temperature and pressure is highly influenced by the nature of the carbonyl compound employed, steric and electronic effects having a strong influence on the thermal stability of the acid. More steric bulk around the carbonyl tending to favor a lower thermal stability of the acid form. Thus, one can tune the strength of the acid and the temperature of facile decomposition by the selection of the appropriate carbonyl compound.

The alcohol can comprise at least one alcohol selected from methanol, ethanol, propanol, butanol, hexanol, heptanol, octanol, nonanol, or decanol. Methanol is preferred for producing fatty acid methyl ester (FAME).

In some embodiments, the reactions described below are carried out in any system of suitable design, including systems comprising continuous-flow (such as CSTR and plug flow reactors), batch, semi-batch or multi-system vessels and reactors and packed-bed flow-through reactors. For reasons strictly of economic viability, it is prefferable that the invention is practiced using a continuous-flow system at steady-state equilibrium.

The figure shows an embodiment of the present invention **100** for the direct production of fatty acid esters from microbial biomass. In this embodiment, microbial biomass **10** is introduced into an acid treatment system **20** containing α-hydroxysulfonic acid where the microbial biomass is allowed to contact with a solution containing an alcohol (provided via **12**) and at least one α-hydroxysulfonic acid thereby producing acid-treated biomass **22** containing at least one fatty acid ester. The acid treatment system may comprise a number of components including *in situ* generated α-hydroxysulfonic acid. The term "in situ" as used herein refers to a component that is produced within the overall process; it is not limited to a particular reactor for production or use and is therefore synonymous with an in process generated component. The term "in situ" is also used to describe forming fatty acid ester in combination with extraction of microbial oil. The acid treated biomass **22** from **20** is introduced to acid removal system **30** where the acid is removed (optionally in its component form) **34** then is recovered (and optionally scrubbed **36**) and recycled (as components, in its combined and/or recombined form) via recycle stream **38** to **20** and the acid treated biomass product stream **32** containing the acid treated biomass substantially free of the alpha-hydroxysulfonic acids is provided to the fatty acid recovery zone **40.** In the fatty acid recovery zone **40,** fatty acid esters are recovered **42** from the treated biomass product stream, and residual **44** is removed. In the recycling of the removed acid, optionally additional carbonyl compounds, SO2, and water may be added as necessary (collectively **38**). The removed acid as components may be recycled to **38** as components and/or in its recombined form(s).

Thus, a typical acid treatment mixture contains (a) a microbial biomass containing at least one lipid, (b) at least one α-hydroxysulfonic acid, (c) an alcohol and (d) water. The process of the invention is effective to directly convert the neutral lipids such as triacylglycerides (TAG) to fatty acid alkyl esters. Thus, in a typical reaction product mixture (or stream), the mixture contains (a) biomass residue (disrupted or acid treated biomass), (b) an alcohol, (c) at least one α-hydroxysulfonic acid, (d) water, and (e) at least one fatty acid alkyl ester. The alkyl group of the fatty acid alkyl ester will be the corresponding alkyl group from the alcohol. For example, the alkyl group of the fatty acid alkyl ester will be methyl group when methanol is used as the alcohol (FAME), ethyl group when ethanol is used (FAEE). The process converts essentially all of the neutral lipids or at least about 60 weight % of the total lipids (including polar lipids and neutral lipids).

Various factors affect the cell disruption of the microbial biomass and the transesterificaton reaction. The carbonyl compound or incipient carbonyl compound (such as trioxane) with sulfur dioxide and water should be added to in an amount and under conditions effective to form alpha- hydroxysulfonic acids. The temperature and pressure of the acid treatment should be in the range to form alpha-hydroxysulfonic acids and to disrupt the microbial biomass cells and to catalyze the transesterification reaction. The amount of carbonyl compound or its precursor and sulfur dioxide should be to produce alpha- hydroxysulfonic acids in the range from about 1 wt%, preferably from about 5 wt%, most preferably from about 10 wt%, to about 55 wt%, preferably to about 50 wt%, more preferably to about 40 wt%, based on the total solution. For the reaction, excess sulfur dioxide is not necessary, but any excess sulfur dioxide may be used to drive the equilibrium in eq. 1 to favor the acid form at elevated temperatures. The microbial biomass to alcohol ratio is preferably in the range of from about 1:5 to about 5:1, based on weight.

The contacting conditions of the hydrolysis reaction may be conducted at temperatures preferably at least from about 50 °C depending on the alpha-hydroxysulfonic acid used, although such temperature may be as low as room temperature depending on the acid and the pressure used. The contacting condition of the hydrolysis reaction may range preferably up to and including about 160 °C depending on the alpha-hydroxysulfonic acid used. In a more preferred condition the temperature is at least from about 80°C, most preferably at least about 100°C. In a more preferred condition the temperature range up to and including about 120°C to about 150 °C The reaction is preferably conducted at as low a pressure as possible, given the requirement of containing the excess sulfur dioxide. The reaction may also be conducted at a pressure as low as about 1 barg, preferably about 4 barg, to about pressure of as high as up to 10 barg The temperature and pressure to be optimally utilized will depend on the particular alpha-hydroxysulfonic acid chosen and optimized based on economic considerations of metallurgy and containment vessels as practiced by those skilled in the art.

The temperature of the acid treatment can be chosen so that the maximum amount of fatty acid alkyl esters is produced from the microbial biomass while limiting the formation of degradation products. The amount of acid solution to "dry weight" biomass determines the ultimate concentration of fatty acid esters obtained. Thus, as high a biomass concentration as possible is desirable.

In some embodiments, a plurality of vessels may be used to carry out the acid treatment. These vessels may have any design capable of carrying out a acid treatment. Suitable vessel designs can include, but are not limited to, batch, trickle bed, co-current, counter-current, stirred tank, or fluidized bed reactors. Staging of reactors can be employed to arrive the most economical solution. Suitable reactor designs can include, but are not limited to, a backmixed reactor (e.g., a stirred tank, a bubble column, and/or a jet mixed reactor) may be employed if the viscosity and characteristics of the partially digested bio-based feedstock and liquid reaction media is sufficient to operate in a regime where bio-based feedstock solids are suspended in an excess liquid phase (as opposed to a stacked pile digester). It is also conceivable that a trickle bed reactor could be employed with the microbial biomass present as the stationary phase and a solution of α-hydroxysulfonic acid passing over the material.

The residual alpha-hydroxysulphonic acid can be removed by application of heat and/or vacuum from the acid treated biomass to reverse the formation of alpha-hydroxysulphonic acid to its starting material to produce a stream containing the acid-treated biomass substantially free of the α-hydroxysulfonic acid. In particular, the product stream is substantially free of alpha-hydroxysulphonic acid, meaning no more than about 2wt% is present in the product stream, preferably no more than about 1wt%, more preferably no more than about 0.2wt%, most preferably no more than about 0.1 wt% present in the product stream. The temperature and pressure will depend on the particular alpha-hydroxysulphonic acid used and minimization of temperatures employed are desirable to preserve the products obtain in the treatment reactions. Typically the removal may be conducted at temperatures in the range from about 50 °C, preferably from about 80 °C, more preferably from 90 °C, to about 110 °C, up to about 150 °C The pressure in the range of from about 0.1 bara to about 3 bara, more preferably from 1 bara (atmospheric) to about 2 bara. It can be appreciated by a person skill in the art that the treatment reaction **20** and the removal of the acid **30** can occurred in the same vessel or a different vessel or in a number of different types of vessels depending on the reactor configuration and staging as long as the system is designed so that the reaction is conducted under condition favorable for the formation and maintainence of the alpha-hydroxysulfonic acid and removal favorable for the reverse reaction. As an example, the reaction in the reactor vessel **20** can be operated at approximately 100 °C and a pressure of 4 barg in the presence of alpha-hydroxyethanesulfonic acid and alcohol and the removal vessel **30** can be operated at approximately 110 °C and a pressure of 0.5 barg. It is further contemplated that the reversion can be favored by the reactive distillation of the formed alpha-hydroxysulfonic acid. In the recycling of the removed acid, optionally additional carbonyl compounds, SO₂, and water may be added as necessary. The process of the invention produces fatty acid esters (fatty acid alkyl esters) in a single step and allows facile removal of the acid catalyst.

While the invention is susceptible to various modifications and alternative forms, specific embodiments thereof are shown by way of examples herein described in detail. It should be understood, that the detailed invention is defined by the appended claims. The present invention will be illustrated by the following illustrative embodiments.

### ILLUSTRATIVE EMBODIMENTS

### General Methods and Materials

In the examples, the aldehyde or aldehyde precursors were obtained from Sigma-Aldrich Co.

Commercial microalgae products obtained from Reed Mariculture Inc. were used to perform the experiments (*Nannochloropsis* green algae).

### Analytical methods

### Lipid determination for bulk algae material:

The determination of the total lipid content (includes FAME) was carried out by using the Dinoex Solvent Extractor (ASE 350). The algae sample was freeze dried over night. Then filled an extractor cell (66 ml) with one gram of algae sample along with sand. Two glass fiber filters (0.2 microns) at both ends of the ASE extractor cell in order to block any potential algae slippage to the extracted solvents. A mixture of methanol and chloroform (65%:35%) was used a solvent system to extract the lipids in 10 min static time at 60°C under 1500 psi pressure. After the ASE extraction, any salts in the extract were washed out in a separatory funnel by shaking with deionized water. Separated chloroform/methanol solvents were evaporated to dryness in Genevac centrifugal evaporator. Lipid content was calculated after weighting the dry lipids using an analytical balance.

The lipid content is reported as Lipid = (sample extract weight - blank extract weight)/ dry weight.

### Examples

### General Procedure for the formation of α-hydroxysulfonic acids.

Aldehydes and ketones will readily react with sulfur dioxide in water to form α-hydroxy sulfonic acids according to the equation 1 above. These reactions are generally rapid and somewhat exothermic. The order of addition (SO₂ to carbonyl or carbonyl to SO₂) did not seem to affect the outcome of the reaction. If the carbonyl is capable of aldol reactions, preparation of concentrated mixtures (> 30% wt.) are best conducted at temperatures below ambient to minimize side reactions. We have found it beneficial to track the course of the reaction using *in situ* Infrared Spectroscopy (ISIR) employing probes capable of being inserted into pressure reaction vessels or systems. There are numerous manufacturers of such systems such as Mettler Toledo Autochem's Sentinal probe. In addition to being able to see the starting materials: water (1640 cm⁻¹), carbonyl (from approx. 1750 cm⁻¹ to 1650 cm⁻¹ depending on the organic carbonyl structure) and SO₂ (1331 cm⁻¹), the formation of the α-hydroxysulfonic acid is accompanied by the formation of characteristic bands of the SO₃⁻ group (broad band around 1200 cm⁻¹) and the stretches of the α-hydroxy group (single to mutiple bands around 1125 cm⁻¹). In addition to monitoring the formation of the α-hydroxy sulfonic acid, the relative position of the equilibrium at any temperature and pressure can be readily assessed by the relative peak heights of the starting components and the acid complex. The definitive presence of the α-hydroxy sulfonic acid can also be confirmed with the ISIR.

### Example 1.

### Formation of 40 % wt. α-hydroxyethane sulfonic acid from acetaldehyde.

Into a 12 ounce Lab-Crest Pressure Reaction Vessel (Fischer-Porter bottle) was placed 260 grams of nitrogen degassed water. To this was added 56.4 grams of acetaldehyde via syringe with stirring. The acetaldehyde/water mixture showed no apparent vapor pressure. The contents of the Fischer-Porter bottle were transferred into a chilled 600 ml C276 steel reactor fitted with SiComp IR optics. A single ended Hoke vessel was charged with 81.9 grams of sulfur dioxide was inverted and connected to the top of the reactor. The SO₂ was added to the reaction system in a single portion. The pressure in the reactor spiked to approximately 3 bar and then rapidly dropped to atmospheric pressure as the ISIR indicated the appearance and then rapid consumption of the SO₂. The temperature of the reaction mixture rose approximately 31 °C during the formation of the acid (from 14 °C to 45 °C). ISIR and reaction pressure indicated the reaction was complete in approximately 10 minutes. The final solution showed an infrared spectrum with the following characteristics: a broad band centered about 1175 cm⁻¹ and two sharp bands at 1038 cm⁻¹ and 1015 cm⁻¹. The reactor was purged twice by pressurization with nitrogen to 3 bar and then venting. This produced 397 grams of a stable solution of 40 % wt. α-hydroxyethane sulfonic acid with no residual acetaldehyde or SO₂. A sample of this material was dissolved in d₆-DMSO and analyzed by ¹³C NMR, this revealed two carbon absorbances at 81.4, and 18.9 ppm corresponding the two carbons of α-hydroxyethane sulfonic acid with no other organic impurities to the limit of detection (about 800:1).

### Examples 2

### In-Situ Microalgae treatment with a-hydroxyethane sulfonic acid solutions.

Into a 300 ml autoclave approximately 40.0 grams of dry Nannochoropsis green algae were placed. To this added 60.0 grams of methanol and 20.0 grams of a 40% aqueous solution of α-hydroxyethane sulfonic acid (prepared from acetaldehyde) with stirring. The reaction mixture was stirred at 1000 to 1500 rpm. The reaction mixture was then heated to the target temperature of 140°C and held for a period of two hours. The heating was discontinued and the reaction cooled to room temperature using a flow of compressed air blowing across the reactor. The reactor was vented and then purged with a slow nitrogen stream for a few minutes to eliminate any sulfur dioxide in the reactor. The reactor was opened and the contents filtered through a medium glass frit funnel using a vacuum flask. The filtrate was dried in a freeze dryer and then extracted with hexane to recover the lipids by Soxhlet extraction. 13.12% of the lipid based on the dry weight was recovered after acid pretreatment, drying, and extraction. A GC-FID analysis of the lipids indicated the presence of monoglyceride (FAME) and no compounds eluting in the diglycerides or triglycerides regions could be detected. The formation of monoglyceride (FAME) was also confirmed by C-13 NMR analysis.

## Claims

1. A method of producing fatty acid esters comprising: (a) providing a microbial biomass; (b) contacting the microbial biomass with a solution containing an alcohol and at least one α-hydroxysulfonic acid thereby producing acid-treated biomass containing at least one fatty acid ester; and (c) recovering said fatty acid ester from the acid-treated biomass.

2. The method according to claim 1, further comprising (d) removing the α-hydroxysulfonic acid in its component form from the acid-treated biomass by heating and/or reducing pressure to produce an acid-removed product containing acid-treated biomass substantially free of the α-hydroxysulfonic acid.

3. The method according to claim 2, further comprising recycling the removed α-hydroxysulfonic acid to step (b) as components or in its recombined form.

4. The method according to any of claims 1 to 3, wherein the α-hydroxysulfonic acid is present in an amount of from 1% wt. to 55% wt., based on the solution.

5. The method according to any of claims 1 to 4, wherein the α-hydroxysulfonic acid is produced from (a) a carbonyl compound or a precursor to a carbonyl compound with (b) sulfur dioxide or a precursor to sulfur dioxide and (c) water.

6. The method according to any of claims 1 to 5, wherein the α-hydroxysulfonic acid is in-situ generated.

7. The method according to any of claims 1 to 6, wherein step (b) is carried out at a temperature within the range of 50 °C to 160°C and a pressure within the range of 1 barg to a10 barg.

8. The method according to any of claims 1 to 7, wherein at least a portion the fatty acid ester is further blended into a biofuel.

9. A composition comprising (a) a microbial biomass containing at least one lipid, (b) at least one α-hydroxysulfonic acid, (c) an alcohol, and (d) water.

10. A composition comprising (a) biomass residue, (b) an alcohol, (c) at least one α-hydroxysulfonic acid, (d) water, and (e) at least one fatty acid alkyl ester.

## Patentansprüche

1. Verfahren zur Herstellung von Fettsäureestern, umfassend: (a) Bereitstellen einer mikrobiellen Biomasse; (b) Inkontaktbringen der mikrobiellen Biomasse mit einer Lösung, die einen Alkohol und mindestens eine α-Hydroxysulfonsäure enthält, wodurch säurebehandelte Biomasse erzeugt wird, die mindestens einen Fettsäureester enthält; und (c) Rückgewinnen des Fettsäureesters aus der säurebehandelten Biomasse.

2. Verfahren nach Anspruch 1, ferner umfassend (d) Entfernen der α-Hydroxysulfonsäure in ihrer Komponentenform aus der säurebehandelten Biomasse durch Erwärmen und/oder Reduzieren des Drucks, um ein Produkt zu erzeugen, aus dem Säure entfernt worden ist und das säurebehandelte Biomasse enthält, die im Wesentlichen frei von α-Hydroxysulfonsäure ist.

3. Verfahren nach Anspruch 2, ferner umfassend das Rückführen der entfernten α-Hydroxysulfonsäure zu Schritt (b) als Komponenten oder in ihrer rekombinierten Form.

4. Verfahren nach einem der Ansprüche 1 bis 3, wobei die α-Hydroxysulfonsäure in einer Menge von 1 Gew.-% bis 55 Gew.-%, bezogen auf die Lösung, vorliegt.

5. Verfahren nach einem der Ansprüche 1 bis 4, wobei die α-Hydroxysulfonsäure aus (a) einer Carbonylverbindung oder einer Vorstufe einer Carbonylverbindung mit (b) Schwefeldioxid oder einer Vorstufe von Schwefeldioxid und (c) Wasser hergestellt wird.

6. Verfahren nach einem der Ansprüche 1 bis 5, wobei die α-Hydroxysulfonsäure in situ erzeugt wird.

7. Verfahren nach einem der Ansprüche 1 bis 6, wobei Schritt (b) bei einer Temperatur im Bereich von 50 °C bis 160 °C und bei einem Druck im Bereich von 1 barg bis 10 barg ausgeführt wird.

8. Verfahren nach einem der Ansprüche 1 bis 7, wobei mindestens ein Teil des Fettsäureesters weiter in einen Biokraftstoff eingemischt wird.

9. Zusammensetzung, umfassend (a) eine mikrobielle Biomasse, die mindestens ein Lipid enthält, (b) mindestens eine α-Hydroxysulfonsäure, (c) einen Alkohol und (d) Wasser.

10. Zusammensetzung, umfassend (a) einen Biomassenrest, (b) einen Alkohol, (c) mindestens eine α-Hydroxysulfonsäure, (d) Wasser und (e) mindestens einen Fettsäurealkylester.

## Revendications

1. Procédé de production d'esters d'acides gras comprenant : (a) la fourniture d'une biomasse microbienne ; (b) la mise en contact de la biomasse microbienne avec une solution contenant un alcool et au moins un acide α-hydroxysulfonique, produisant ainsi une biomasse traitée à l'acide contenant au moins un ester d'acide gras ; et (c) la récupération dudit ester d'acide gras à partir de la biomasse traitée à l'acide.

2. Procédé selon la revendication 1, comprenant en outre (d) l'élimination de l'acide α-hydroxysulfonique sous sa forme constitutive de la biomasse traitée à l'acide en chauffant et/ou en réduisant la pression pour produire un produit éliminé à l'acide contenant une biomasse traitée à l'acide sensiblement exempte de l'acide α-hydroxysulfonique.

3. Procédé selon la revendication 2, comprenant en outre le recyclage de l'acide α-hydroxysulfonique éliminé à l'étape (b) en tant que composants ou sous sa forme recombinée.

4. Procédé selon l'une quelconque des revendications 1 à 3, dans lequel l'acide α-hydroxysulfonique est présent en une quantité de 1 % en poids à 55 % en poids, sur la base de la solution.

5. Procédé selon l'une quelconque des revendications 1 à 4, dans lequel l'acide α-hydroxysulfonique est produit à partir (a) d'un composé carbonyle ou d'un précurseur d'un composé carbonyle avec (b) du dioxyde de soufre ou un précurseur du dioxyde de soufre et (c) de l'eau.

6. Procédé selon l'une quelconque des revendications 1 à 5, dans lequel l'acide α-hydroxysulfonique est généré in situ.

7. Procédé selon l'une quelconque des revendications 1 à 6, dans lequel l'étape (b) est réalisée à une température dans la plage de 50 °C à 160 °C et à une pression dans la plage de 1 barg à 10 barg.

8. Procédé selon l'une quelconque des revendications 1 à 7, dans lequel au moins une partie de l'ester d'acide gras est en outre mélangée dans un biocarburant.

9. Composition comprenant (a) une biomasse microbienne contenant au moins un lipide, (b) au moins un acide α-hydroxysulfonique, (c) un alcool et (d) de l'eau.

10. Composition comprenant (a) un résidu de biomasse, (b) un alcool, (c) au moins un acide α-hydroxysulfonique, (d) de l'eau et (e) au moins un ester alkylique d'acide gras.
